(19) Europäisches Patentamt / European Patent Office / Office européen des brevets

(11) **EP 2 878 315 A1**

(12) **EUROPEAN PATENT APPLICATION**

(43) Date of publication:
03.06.2015 Bulletin 2015/23

(51) Int Cl.:
*A61L 27/18* (2006.01)    *A61L 27/54* (2006.01)
*A61L 27/56* (2006.01)

(21) Application number: 14195230.9

(22) Date of filing: 27.11.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 28.11.2013 PCT/IB2013/002899

(71) Applicant: **Heart Biotech Nano Limited**
**London WS1 1YN (GB)**

(72) Inventors:
• **Sohier, Jérôme Jean-Luc**
**30150 Saint Géniès de Comolas (FR)**
• **Yacoub, Magdi Habib**
**London, W5 2JL (GB)**

(74) Representative: **Williams, Gareth Owen**
**Marks & Clerk LLP**
**62-68 Hills Road**
**Cambridge**
**CB2 1LA (GB)**

(54) **Artificial tissue**

(57)    A method of forming a matrix of aligned nanofibres of elevated pore size and porosity comprises spraying a polymer solution towards a rotating drum so as to form nanofibres which are collected on the drum. The matrix can be used to form artificial tissue by removing the matrix from the drum, depositing cells onto the matrix and allowing the cells to form artificial tissue. Such artificial tissue finds use in the treatment of disease or damaged tissue, and in particular in the treatment of cardiovascular disease.

Figure 1

**Description**

The present invention relates to a method of forming artificial tissue, and tissue produced thereby. In particular, the invention relates to the formation of artificial tissue using a matrix of aligned nanofibres.

**Introduction**

Despite years of continuous progress in medicine and surgery, cardiovascular diseases are still a leading cause of death worldwide. Among these pathologies, heart valve disease represents an important part of cardiovascular surgical procedures. Although prosthetic valve replacement is a common and efficient procedure, a need for alternative solutions exists, especially for children who require valve growth and therefore a living prosthetic. Tissue engineering, which aims to create new and functional living tissues by the *in vitro* association of supportive structure (scaffolds) and cells, has great potential to provide such advanced prostheses.

Three-dimensional biomaterial scaffolds that support cell infiltration and tissue organization have been extensively investigated for applications in regenerative medicine and tissue engineering. Closely mimicking the extracellular matrix (ECM) at the nanometer level is still, however, a challenge. The ECM is a complex and dynamic environment secreted by cells to provide the structure and properties of a tissue. Aside from proteoglycans, the main component of the ECM is fibrillar collagen, which serves as a mechanical framework, a point of anchor for cells, and provides regulatory signals. The organization of the ECM is increasingly recognized for its cell-instructive role through cell shape, focal adhesion dynamics and resulting mechano-transduction signals.

Collagen-based biomaterials for use in tissue engineering are commercially available. Typically, commercial collagens are extracted from a mammalian source, decellularized, purified and sterilized. However, the use of mammalian-derived collagen presents potential hazards, including contagion through the transmission of pathogens. It is therefore desirable to engineer artificial tissues using synthetic scaffolds which provide the cells with a structure similar to natural collagen networks.

Methods have been sought to produce polymeric fibres which mimic more closely the ECM. Optimally, the synthetic mimetic scaffold should provide cells with a structure similar to native collagen networks in terms of its organization and properties. Although synthetic fibrillar matrices are known, cellularization remains problematic.

It is known to produce nanofibres by electrospinning. However, this method is complex and requires high electric fields. Furthermore, a significant limitation of electrospinning is the production of structures composed of closely packed nanofibers and hence small porosity and pore sizes (for example, pore sizes of a few $\mu$m) [1-4]. As a result, cellular infiltration and colonization is hampered and limited to the surface of electrospun matrices [5, 6]. To circumvent this limitation, various complex ways have been described to improve cell penetration, relying on the use of flow perfusion to force the cells infiltration [1, 2] or on the development of composites with sacrificial content to improve cellular migration, which then requires 12 weeks of culture [7] The alignment of the nanofibers further aggravates this drawback [8] and reduces the capacities of the matrices to direct the formation of an anisotropic tissue.

WO 2010/081832 describes jet-spraying polymer solutions to form an interlaced nanofibre web without preferential organization of the nanofibers. Such nanofibres webs have relatively small pore diameters, making it more difficult for cells to colonize the web.

US2007/0269481 describes the use of electrospinning to form a nanofibre polymer composition having pore sizes on the order of 4 $\mu$m.

The present invention has been devised with these issues in mind, to produce nanofibrillar structures of high porosity and pore size in a straightforward procedure.

**Summary of the invention**

According to a first aspect of the invention, there is provided a method of forming an artificial tissue having high porosity and pore size, the method comprising:

> forming a matrix of aligned nanofibres by spraying a polymer solution towards a rotating drum so as to form nanofibres which are collected on the drum;
> removing the matrix from the drum; and
> depositing cells onto the matrix of aligned nanofibres and allowing the cells to form artificial tissue.

Importantly, the method of the invention does not rely on electrospinning to produce the matrix; which, as described above, produces matrices with small pore size and low porosity. In preferred embodiments of the invention, the spraying step comprises placing a polymer solution under pressure to eject it through a nozzle. The pressure may be gas pressure. The solution may have a viscosity above a predetermined shear viscosity, as described in WO2010/081832.

According to a second aspect of the invention, there is provided an artificial tissue comprising a matrix of aligned nanofibres. The matrix provides a biomimetic scaffold which acts as a substrate for cellular colonization. The artificial tissue may be obtainable by the method of the first aspect.

The anisotropic organization of the collagen/elastin in some tissues contributes to the mechanical properties of the tissue. In the context of heart valves, the collagen organization is thought to be of particular importance to ensure physiological compliance and mechanical properties. In heart valves, the collagen fibres are arranged anisotropically along the circumferential direction of the valves cusps. In the pericardium, the structural features and organization of collagen 1 fibers confer a non-linear and anisotropic mechanical behavior [9, 10]. The present inventors have recognized the importance of this anisotropic organization, and believe that reproducing this structure will allow artificial tissue to more closely mimic the native properties of the heart valve or pericardium, while providing a suitable environment for the synthesis of a functional valve or pericardial tissue by cells. It has been found that by spraying a polymer solution onto a rotating drum, a matrix can be produced in which the nanofibres are aligned in the direction of rotation. The matrix of aligned nanofibres is believed to mimic the native anisotropy of heart valves and other tissues such as pericardium.

According to a third aspect of the present invention, there is provided the use of the artificial tissue of the second aspect of the invention for producing an artificial heart valve or artificial pericardium.

According to a fourth aspect of the present invention, there is provided the artificial tissue of the second aspect of the invention for use in therapy or surgery.

According to a fifth aspect of the present invention, there is provided the artificial tissue of the second aspect of the invention for use in the treatment of diseased or damaged tissue.

According to a sixth aspect of the present invention, there is provided a method of treating diseased or damaged tissue, said method comprising administering the artificial tissue of the second aspect of the invention to a patient in need thereof. The artificial tissue may be administered by implanting the artificial tissue into a patient (for example to replace existing tissue), or grafting the artificial tissue onto the patient (for example as a skin grant).

In particular, the diseased or damaged tissue may have anisotropic organization.

The artificial tissue may be used for the treatment of diseased or damaged tissue including skin (e.g. bed sores, hypertrophic scarring, burns), ligaments (e.g. ligament inflammation and rupture), tendons (e.g. inflammation, rupture, inherited diseases such as Ehlers-Danlos syndrome, Marfani syndrome or homocystinuria), vessels (e.g. aneurisms, arteriosclerosis, atherosclerosis, vessel grafts), valves (e.g. heart valves) pericardium (e.g. pericarditis, pericardial effusion, cardiac tamponade, and constrictive pericarditis), and/or muscles (e.g. tumours). In particular, the artificial tissue of the invention may find use in the treatment of cardiovascular disease, for example heart valve disease or pericardial disease. The patient may be animal or human.

The present invention also resides in a method of forming a matrix of aligned nanofibres of high porosity and pore sizes, said method comprising spraying a polymer solution towards a rotating drum so as to form nanofibres which are collected on the drum, and removing the matrix from the drum. The matrix is preferably suitable for use in the preparation of artificial tissue, by providing a swift and efficient cellularization.

The invention further resides in a matrix of aligned nanofibres. The matrix of aligned nanofibres may be obtainable by the methods described herein. The nanofibres forming the matrix may be biocompatible so that the matrix is suitable for the preparation of artificial tissue.

The following statements may apply to any of the above-mentioned aspects of the invention, as appropriate.

The diameter of the rotating drum has been found to influence the alignment of the nanofibre matrix. The diameter of the drum may be from 50 mm to 2 m. In some embodiments, the diameter of the drum is at least 50mm, at least 100 mm, at least 120 mm or at least 135 mm, for example 150 mm. In further embodiments, the diameter of the drum may be no more than 1 m, no more than 50 cm, or no more than 30 cm. The use of a drum of at least 100 mm in diameter facilitates the formation of homogeneously aligned fibres.

The degree of alignment of the nanofibres has been found to be a function of the rotation speed of the drum. The speed of rotation may be from 8 m/s to 100 m/s, preferably 10 m/s to 50 m/s. In some embodiments, the drum is rotated at a speed of at least 10 m/s, at least 15 m/s or at least 20 m/s. In further embodiments, the drum is rotated at a speed of from 20 to 30 m/s, for example approximately 25 m/s. If the speed of rotation is too low, the fibres will not be aligned, but if the speed is too high the productivity is decreased.

The porosity and pore size are important properties of an artificial nanofibrillar matrix, since these factors dictate the ability of cells to penetrate and colonize the matrix. Surprisingly, the pore size and porosity of the matrix has also been found to be a function of the speed of rotation of the drum. The present invention permits the manufacture of products having high porosity and pore size, which are suitable for use as artificial tissues. By "high porosity and pore size" is meant porosity and pore size falling within the ranges disclosed in the following two paragaphs.

The porosity of the matrix is at least 95%, and preferably may be from 95 to 99.5%. In some embodiments, the porosity of the matrix (as measured by the method described in Example 1) is at least 96%, at least 97%, at least 98%, at least 98.5% or at least 99%.

The median pore size of the matrix is at least 50 $\mu$m, and preferably may be from 50 to 110 $\mu$m. In some embodiments, the pore size (as measured by the method described in Example 1) is at least 60 $\mu$m, at least 70 $\mu$m, at least 80 $\mu$m or at least 90 $\mu$m. A matrix which can be more easily colonized by cells is thought to be beneficial for the formation of homogeneous tissue.

The artificial tissue of the invention may comprise the matrix of aligned nanofibres colonized with cells. The tissue may be any tissue with known anisotropy. Examples of such tissues include valves, tendons, ligaments, skin, vessels and muscles. Cells that may be used to colonize the artificial tissue include, but are not limited to, adipose-derived mesenchymal stem cells, adipose-derived stem cells, fibroblasts, smooth muscle cells, endothelial cells, bone marrow derived stem cells, valve interstitial cells and myofibroblasts. The cells may be human or animal. In some preferred embodiments, the artificial tissue is artificial cardiac tissue. In these embodiments, the cells are cardiac cells, such as valve interstitial cells.

The matrix of aligned nanofibres may be functionalized with bioactive molecules, for example, to aid cellular colonization. In some embodiments, the method further comprises functionalizing the matrix by binding bioactive molecules to the nanofibres. This may also be described as 'decorating' the matrix. The functionalization of nanofibrillar matrices may be achieved through the formation of covalent or non-covalent bonds.

The bioactive molecules may be peptides. The peptides may comprise or consist of short chains of amino acids, for example from 3 to 10 amino acids. In preferred embodiments, the peptides are capable of binding to cell receptors such as integrins. Examples of peptides that may be used include KQAGDV, LDV, IDS RLD, KRLDGS, RGD, IET, YYGDLR, FYFDLR, YIGSR, REDV, YKVAV, RNIAEIIKDI, KHIFSDDSSE, VPGIG, FHRRIKA, and KRSR.

The bioactive molecules may be attached to the matrix via a linker. The linker may be attached to the bioactive molecule prior to functionalization of the matrix or, alternatively, the linker may be attached to the matrix prior to binding the bioactive molecules to the linker. Thus, in some embodiments, the method comprises functionalizing the matrix with linker-bioactive molecule conjugates. In other embodiments, the step of functionalizing the matrix comprises binding linker molecules to the matrix, and then binding the bioactive molecules to the linkers. Examples of suitable linkers include dendritic polymers (also known as "dendrimers" or "dendronized polymers"), in particular poly-ionic dendritic polymers (e.g. poly(amidoamine), poly(ethylenimine), or linear polymers (e.g. poly(acrylic acid)). The linkers may be grafted with a peptide of interest prior to functionalization of the matrix.

**Brief description of the Figures**

Embodiments of the invention will now be described by way of example and with reference to the accompanying Figures in which:

Figure 1 is a schematic diagram of apparatus which may be used to form a matrix of aligned nanofibres in accordance with an embodiment of the present invention;
Figures 2a to 2e are a series of graphs showing the correlation between fibre anisotropy and rotation speed for matrices in accordance with n embodiment of the present invention;
Figures 3a and 3b are graphs showing the mechanical properties of the aligned nanofibre matrices as a function of rotation speed. Young's modulus (Figure 3a) and ultimate tensile strength (Figure 3b) were measured longitudinally and orthogonally to the drum rotation direction. #, $, § and * denote a statistically significant difference ($p < 0.05$) between Young's modulus and ultimate tensile strength measured respectively longitudinally and orthogonally ($n = 5 \pm$ standard deviation);
Figures 4a, 4b and 4c are graphs showing the macroscopic properties of the aligned nanofibre matrices as a function of rotation speed. Matrices void fraction (Figure 4a), median pore diameter (Figure 4b) and total pore area (Figure 4c) were measured as a function of drum rotation speed. + indicates a statistical difference between porosities ($n = 5 \pm$ standard deviation);
Figure 5 is a picture illustrating the improved cellularization of aligned nanofibre matrices at day one, day ten, and day twenty, as compared to non-aligned nanofibre matrices
Figure 6a is a graph showing the effect of RGD decoration of an aligned nanofibre matrix on cellular attachment; and
Figure 6b is a graph showing the effect of RGD decoration of an aligned nanofibre matrix on cell spreading.

**Detailed description of the invention**

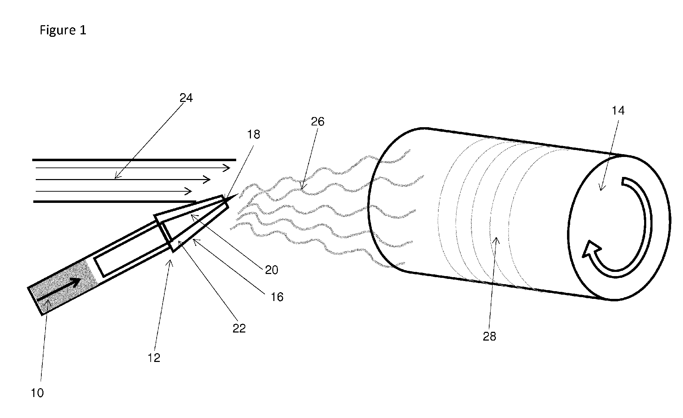

With reference to Figure 1, a matrix of aligned nanofibres may be produced by spraying a polymer solution (10) through a spray nozzle (12) under gas pressure (i.e. jet-spraying), in the direction of a rotating drum (14). The spray nozzle (12) comprises a conical tube (16) having an outlet (18) and containing a conical needle (20). A passageway (22) is defined between the conical tube (16) and the needle (20). The polymer solution (10) is aspirated into the passageway (22), and exits the nozzle (12) through the outlet (18) in front of a gas stream (24) (e.g. compressed air).

The solution (10) is diffracted and nanofibres (26) are formed in transit by cohesion of the polymer phase and solvent evaporation. The nanofibres (26) collected on the drum (14) as it rotates, thereby forming a matrix of aligned nanofibres (28).

The drum comprises a cylindrical surface on which the nanofibres are collected to form the matrix. Preferably, the cross-section of the cylinder is circular. The collecting surface of the cylinder may be continuous or discontinuous. In some embodiments, the drum comprises a discontinuous collecting surface formed from a plurality of wires extending parallel to a central axis of the drum, the wires being spaced at regular intervals equidistant from and around the central axis. This arrangement has been found to be particularly effective for obtaining aligned fibres. The plurality of wires may extend between a pair of opposing discs arranged on the central axis.

The collecting surface of the drum may be coated with, or made from, a non-stick material such as PTFE (polytetrafluoroethylene). The use of a non-stick material or coating facilitates removal of the nanofibre matrix from the drum after spraying.

By "aligned" it will be understood that at least 50% of the nanofibres of the matrix have an orientation which does not diverge by more than 20 degrees (positive or negative) from the average (i.e. median) orientation. In some embodiments, at least 55%, at least 60%, at least 65% or at least 70% of the nanofibres have an orientation which does not diverge more than 20 degrees (positive or negative) from the average orientation.

The term "nanofibre", as used herein, will be understood as meaning fibres having an average (i.e. mean) diameter of less than 1200 nm. In some embodiments, the fibres have an average diameter of from 300 to 1100 nm, e.g. approximately 600 nm. During production of the matrix, bundles of nanofibres may be formed, which can resulting in microfibers of up to 10 $\mu$m.

It will be appreciated by those skilled in that art that the parameters of the step of spraying the polymer solution and properties of the solution itself will influence the properties of the resulting matrix and, as such, the artificial tissue produced from the matrix.

The spraying distance (e.g. the distance between the opening of the nozzle and a collecting surface of the rotating drum) may be from 5 to 50 cm, from 10 to 40 or from 20 to 30 cm. It has been found that the spraying distance affects the homogenous nature and the density of the matrix. For example, a spraying distance of 40 cm may allow the formation of aligned fibers in the shape of inhomogeneous big intertwined bundles. A distance of 30 cm may provide homogenous matrices. A distance of 20 cm may provide a biphasic structure with nanofibers over a dense polymer layer. Less than 20 cm may result in a dense polymer membrane.

The pressure of the gas also influences the properties of the matrix. Preferably, the gas pressure (as measured at the gas outlet) is at least $3 \times 10^5$ N/m$^2$ (bars), or at least $5 \times 10^5$ N/m$^2$. The pressure may be no more than $10 \times 10^5$ N/m$^2$. In some embodiments, the pressure is from $6 \times 10^5$ N/m$^2$ to $8 \times 10^5$ N/m$^2$, e.g. $7 \times 10^5$ N/m$^2$. Decreasing the pressure has been found to reduce the productivity and homogeneity of the matrix. Increasing the pressure has been found to result in large bundles of fibres. However, it will be appreciated that the optimal pressure will depend on other factors, such as the shape of the nozzle.

The size of the nozzle opening is also important. If the opening is too large, too may nanofibres will be produced, resulting in reduced productivity. It is preferred that the diameter of the nozzle opening is from 0.4 to 0.8 mm, e.g. approximately 0.6 mm.

Another important parameter which affects the productivity of the method and the homogeneity of the matrix is the height from which the polymer solution is sprayed, relative to the drum. Optimally, the polymer solution is sprayed from a height which is the same height as the central axis of the drum. The nozzle opening may therefore be transversely aligned with the central axis of the drum.

The polymer solution comprises a polymer or a mixture of polymers dissolved in a solvent. The polymer may selected from the group consisting of: polycaprolactone, polyester, (poly(dioxanone), poly(ortho esters), poly(amide esters), poly(anhydrides), polyvinyl esters, (poly(tetrafluoroethylene), poly(ethylene), poly(ethylene glycol), polypropylene oxide, poly(lactic-co-glycolic acid, or mixtures thereof. In some embodiments, the polymer is polycaprolactone. This polymer is advantageous in that it is biocompatible and biodegradable, has good mechanical properties, and a slow rate of degradation.

The solvent may be any solvent suitable for dissolving the chosen polymer. The solvent may be one having a vapour pressure of more than 5.8 kPa. Examples of suitable solvents include chlorinated solvents, tetrahydrofuran, diethyl ether, alcohols and fluorinated alcohols (e.g. hexafluoroisopropanol).

The polymer solution may have a viscosity of above a predetermined shear viscosity value at room temperature. The predetermined shear viscosity may be equal to 0.068 Pa.s. for a shear rate increasing from 0.05 to 9000 s$^{-1}$ in 60s. In some embodiments, the shear viscosity of the polymer solution is above 0.1 Pa.s. for a shear rate increasing from 0.05 to 9000 s$^{-1}$ in 60s.

The matrix may be functionalized with bioactive molecules as required. "Bioactive molecule", as used herein, will be understood to mean a molecule which is capable of performing a biological function. Examples of such biological functions include, but are not limited to, anchoring cells, providing or triggering cellular signals and regulating cellular functions. Bioactive molecules may be bound to the matrix through covalent or non-covalent interactions. It will be appreciated by those skilled in the art that the type of interactions, and the chemistry involved, will depend on both the type of bioactive molecule and the properties of the nanofibres, in particular on the functional groups available for binding.

An example covalent strategy uses succinimide/carbodiimide chemistry to bind carboxylic acid groups of the nanofibres (when present) with available primary amines (e.g. lysines) of bioactive molecules.

Matrices of aligned nanofibres may be decorated non-covalently using dendritic polymers which, optionally, may be grafted with a peptide of interest. Functionalization may be achieved by immersing the matrix in a dendritic polymer solution for a period of time sufficient to effect binding.

Other methods of functionalizing polymer matrices with bioactive molecules via covalent or non-covalent interactions will be known to those skilled in the art.

In some instances, the success of the functionalization depends on the hydrophobicity of the polymer used to form the nanofibre matrix. To facilitate homogeneous functionalization, the method may further comprise treating the matrix to modify its wettability, prior to functionalizing the matrix with bioactive molecules. The treatment may comprise gas plasma treatment (e.g. oxygen plasma for 10 minutes at 50 watt, 40 kHz) or ethanol treatment (e.g. immersing the matrix in 70% ethanol for at least one hour at room temperature). It will be appreciated that the type of treatment used will depend on the type of polymer. For example, ethanol treatment would not be suitable for treating an alcohol-soluble polymer. For gas plasma treatment, the length and power of the treatment must be selected to avoid melting of the nanofibres.

The artificial tissue of the invention may comprise the matrix of aligned nanofibres colonized with cells. Thus, the step of allowing the cells to form artificial tissue may comprise culturing the cells which have been deposited onto the matrix. Suitable conditions and reagents for cell culture will depend on the type of cell, and will be known to those skilled in the art. The culture conditions may comprise an atmosphere of 5% $CO_2$, 95% air. The cells may be cultured at 37 °C. The functionalized matrix of aligned nanofibres with the cells deposited thereon may be placed in a bioreactor for culturing the cells. Mechanical stimulations may be applied during the culturing process, for example, to reproduce cardiac flow and pressure.

Methods of treating or preventing diseases or conditions such as cardiovascular disease comprise implanting the artificial tissue of the invention into a patient. The artificial tissue may be used to replace tissue which is damaged or diseased. For example, the artificial tissue may be used to form an artificial heart valve which is used to treat a patient with heart valve disease.

To avoid rejection, it is advantageous to transplant artificial tissue which has been generated using a patient's own cells. The artificial tissue may therefore comprise a matrix of aligned nanofibres colonized with cells derived from a patient that the tissue is intended for. Therefore, in some embodiments, a method of treating a patient may comprise: forming an artificial tissue comprising depositing cells onto a matrix of aligned nanofibres and allowing the cells to form artificial tissue, wherein the cells are derived or previously extracted from the patient; and transplanting the artificial tissue into the patient.

**Example 1: Effect of rotation speed on matrix properties**

**1.1 Formation of matrix of aligned nanofibres**

Nanofibres were produced by spraying a solution of polyaprolactone (PCL) in chloroform (0.1 g/ml) onto a drum (nozzle opening of 0.5 cm, air pressure of 6 bars, spraying distance of 30 cm). The drum was rotated at different speeds and the alignment of the nanofibres in the resulting matrices was quantified using the NIS-Elements software (Nikon Instruments Europe B.V., Badhoevedorp, The Netherlands) "orientation" routine. After thresholding of the SEM images, the software automatically separated each of the selected fibers into smaller objects of comparable sizes while excluding non-linear objects. Longitudinal orientation relative to the picture bottom (referred as 0 degree) of each object was then computed. 150 values were obtained on average for a given picture.

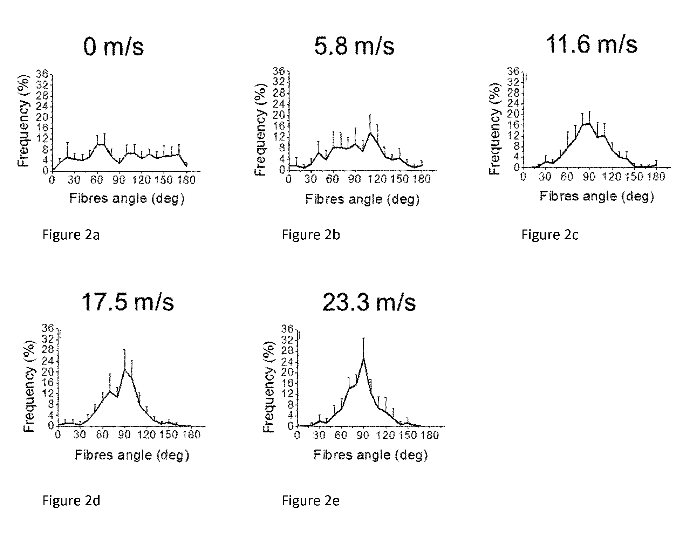

The average fibre diameter produced was 600 nm. The fibre diameter span was 100-1100 nm. As shown in Figures 2a to 2e, the degree of alignment increases with increasing rotation speed.

**1.2 Macroscopic anisotropy**

The Young's modulus and ultimate tensile strength of the matrices were measured longitudinally and orthogonally to the rotation direction of the drum. Mechanical properties of the nanofibrillar mats (5mm x 1 cm, 500-1000 $\mu$m thick) were measured by tensile testing (Bose Electroforce TestBench, Minnesota, USA) at a speed of 0.1 mm/s. For each condition, 5 samples, cut longitudinally and orthogonally to the drum rotational direction, were measured.

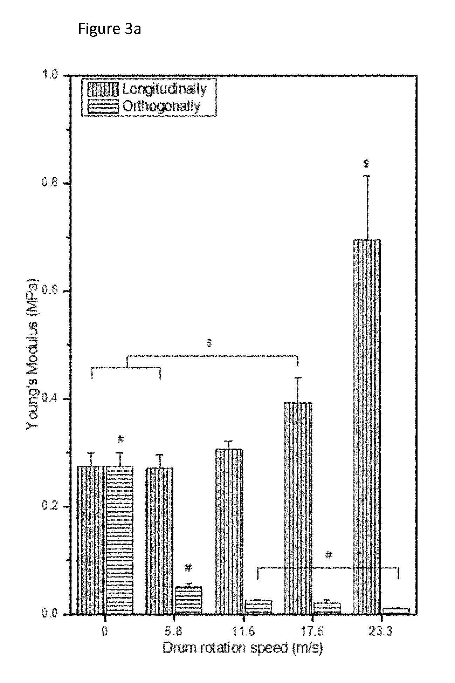

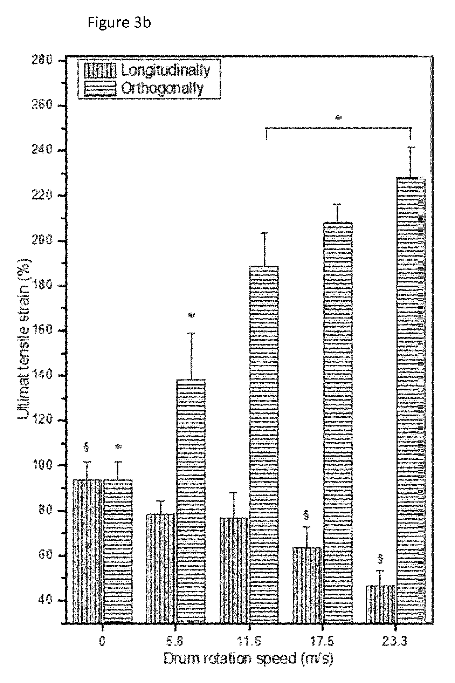

The mechanical properties of the matrices were found to be influenced by the drum rotation speed, and hence by the fibre alignment. It was found that mechanical anisotropy increases with fibrillar alignment (Figures 3a and 3b).

The porosity, pore size and total pore area of the matrices were measured. Porosity of the matrices was evaluated from the dry weight and volume of 5 cylindrically cored samples (12 mm in diameter, 800-1000 $\mu$m thick). The thickness of each sample was determined using a micro-calliper and resulting porosity was then deduced from the following equation:

$$p = 1-(\text{sample weight}/(\text{sample volume x polymer density}))$$

Median pore diameter and total pore area of the matrices were evaluated using mercury intrusion porosimetry (Autopore IV 9500, Micromeritics, Norcross, U.S.A.) as described by Pham QP, Sharma U, Mikos AG. Electrospun poly(epsilon-caprolactone) microfiber and multilayer nanofiber/microfiber scaffolds: characterization of scaffolds and measurement of cellular infiltration. Biomacromolecules. 2006 Oct;7(10):2796-805. Briefly, each measured sample was weighed prior to being filled with mercury from an initial pressure of 0.33 to 60,000 pound per square inch absolute (psia). The pore diameter distribution was then calculated from the Washburn equation.

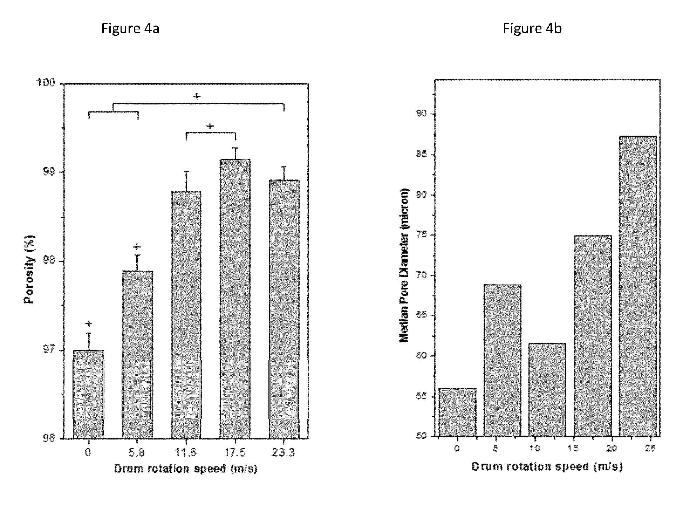

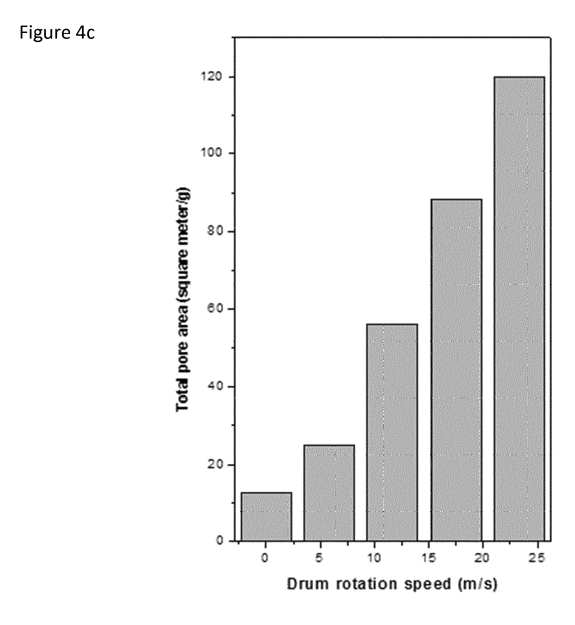

Unexpectedly, the porosity (expressed as void fraction, Figure 4a), median pore diameter (Figure 4b) and total pore area (Figure 4c) were found to be a function of drum rotation speed. Increasing the rotation speed, and thus the alignment of the nanofibres, was also found to result in increased porosity and pore size. Very high porosities (up to 99%) and pore sizes (up to 87 $\mu$m) were achieved. These values are significantly higher than those reported for structures obtained by electrospinning.

### 1.3 Anisotropic matrices as hADSC substrates

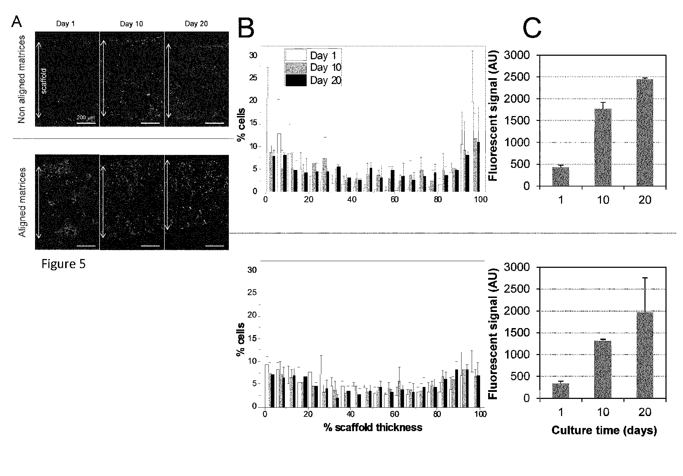

Human adipose-derived stem cells (hADSC) were seeded and cultured on isotropic and anisotropic matrices (n=3, $\Phi$-1 cm, 500-700 $\mu$m thick) for 20 days. The cellular penetration and colonization of the matrices was monitored by cross-section and DAPI staining of cell nuclei. The highly porous aligned nanofibres matrices were found to allow a rapid cellular colonization and proliferation (Figure 5a). Nuclei distribution along the matrices' thickness over time was quantified by image analysis (Figure 5b), while cellular proliferation within the matrices over 20 days was assessed by DNA quantification (Figure 5c). The highly porous aligned matrices allowed rapid cellular colonization and proliferation. It was found that hADSCs bridge the anisotropic scaffolds more extensively and homogenously, and proliferate more, as compared to isotropic matrices. The anisotropic matrices (i.e. aligned nanofibre matrices) are therefore a suitable substrate for cellular colonization.

Phallodin staining and confocal microscopy analysis of the colonized matrices also revealed that anisotropic (i.e. aligned) matrices are instructional for cell phenotype and organization. The cells cultured on anisotropic matrices were observed to deposit extracellular matrix components, including collagen I, collagen III and tropoelastin (as visualized by immunohistochemistry). Synthesis of these components was observed to be more consistent inside the anisotropic matrices than in isotropic matrices. This demonstrates the functional capacity of hADSCs cultured in highly porous anisotropic matrices, and shows that nanofibrillar matrices allow the production of ECM by the cells.

The expression of surface markers CD44, Stro-1 and Fibroblast surface antigen (FSA) by hADSCs was monitored over the 20-day culture period using standard immunohistochemistry techniques. Consistent expression of CD44 and FSA was observed over the culture time. A decrease in Stro-1 expression was observed, suggesting a change in phenotype. No differentiation of the stem cells towards the osteogenic, chondrogenic or adipogenic mesodermal lineage and no distinct effect of anisotropy was observed. This result indicates that the fibre anisotropy does not induce the differentiation of undifferentiated stem cells.

### 1.4 Influence of anisotropy on human valve interstitial cells

Human valve interstitial cells (hVIC, P3, 500000) were seeded and cultured (DMEM) on isotropic and anisotropic matrices (n=3, $\Phi$-1 cm, 500-700 $\mu$m thick) for 20 days. hVICs were found to behave similarly to hADSCs in anisotropic matrices. Consistent expression of CD44 and FSA was observed over the culture period for both types of matrices. A slight increase of $\alpha$SMA expression was seen in isotropic matrices.

### Conclusions

From the results obtained it can be concluded that the present invention provides anisotropic matrices with improved porosity and pore size, compared to matrices obtained by electospinning. These matrices are suitable for cellular colonization and ECM synthesis. The nanofibrillar anisotropy instructs cell morphology and organization. The matrices are therefore suitable for tissue engineering and formation of valve-like tissues.

**Example 2: Functionalization of aligned nanofibre matrices: covalent strategy**

A matrix of aligned nanofibres can be functionalized with peptides using the following protocol:

The matrix is first oxidized by oxygen plasma for 10 minutes (50 watt, 40 kHz), and then immediately incubated in activation buffer (0.1M (2-[morpholino]ethanesulfonic acid) MES, 0.5M NaCl, pH 6.0) containing 5 mM and 2 mM of respectively Sulpho-NHS and EDC. Unreacted EDC is quenched with 2-mercaptoethanol (20 mM) and activation buffer removed. A 50 $\mu$g/ml peptide solution in phosphate-buffered Saline (PBS, 0.1M sodium phosphate, 0.15M NaCl, pH 5.5) is then added to cover entirely the matrix and incubated overnight at 4°C. Unreacted sulpho-NHS is quenched with glycine (20mM) and the samples are rinsed with PBS three times.

**Example 3: Functionalization of aligned nanofibre matrices with RGD peptide using linkers**

A matrix of aligned nanofibres was produced as described above. RGD peptide was obtained from Peptides International Inc. (Louisville, USA). Dendrimers grafted with RGD peptide were obtained from a third party supplier. The matrix was immersed in a 50 $\mu$g/ml dendrimer solution for 2 hours at room temperature. The matrix was then be rinsed extensively with PBS to removed unbound dendrimers.

After functionalization of the aligned nanofibre matrix with RGD peptide, human telomerase reverse transcriptase (hTERT) immortalized cells (15000/200 $\mu$l) were deposited onto 25 mm$^2$ of the decorated surfaces and incubated in 5% CO2-95% air at 37°C. After 2 hours, the samples were rinsed slowly three times in PBS, fixed in 10% paraformaldehyde, stained with (4',6-Diamidino-2-Phenylindole (DAPI) and phalloidin (Sigma Chem. corp., St. Louis, USA). The matrix was then mounted and observed using a fluorescent microscope. The amount of attached cells and the cell surface was quantified by image analysis. The number of attached cells was obtained by quantifying the number of nucleus observed in four random pictures of untreated and decorated samples. The average cytoplasm surface per cell was obtained by dividing the phalloidin stained surface of each picture by the corresponding cell number.

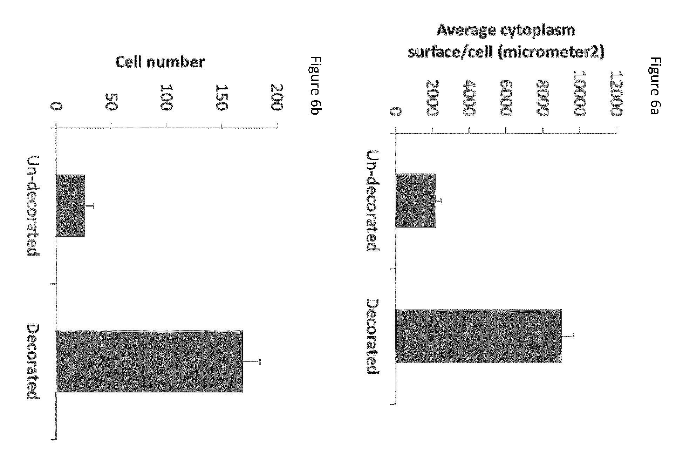

The functionalization of the matrix resulted in a greater than 4-fold increase in cellular attachment (Figure 6a) and a greater than 6-fold increase in cell spreading (Figure 6b), as compared to a non-functionalized matrix.

<u>REFERENCES</u>

1. Ekaputra, A.K., Prestwich, G.D., et al., Combining electrospun scaffolds with electrosprayed hydrogels leads to three-dimensional cellularization of hybrid constructs. Biomacromolecules, 2008. 9(8): p. 2097-2103.

2. Pham, Q.P., Sharma, U., and Mikos, A.G., Electrospun poly(epsilon-caprolactone) microfiber and multilayer nanofiber/microfiber scaffolds: characterization of scaffolds and measurement of cellular infiltration. Biomacromolecules, 2006. 7(10): p. 2796-805.

3. Zhu, X., Cui, W., et al., Electrospun Fibrous Mats with High Porosity as Potential Scaffolds for Skin Tissue Engineering. Biomacromolecules, 2008. 9(7): p. 1795-1801.

4. Hong, J.K. and Madihally, S.V., Three-dimensional scaffold of electrosprayed fibers with large pore size for tissue regeneration. Acta biomaterialia, 2010. 6(12): p. 4734-42.

5. Nisbet, D.R., Forsythe, J.S., et al., Review paper: a review of the cellular response on electrospun nanofibers for tissue engineering. Journal of biomaterials applications, 2009. 24(1): p. 7-29.

6. Martins, A., Araujo, J.V., et al., Electrospun nanostructured scaffolds for tissue engineering applications. Nanomedicine, 2007. 2(6): p. 929-42.

7. Baker, B.M., Shah, R.P., et al., Sacrificial nanofibrous composites provide instruction without impediment and enable functional tissue formation. Proceedings of the National Academy of Sciences of the United States of America, 2012.109(35): p. 14176-81.

8. Moffat, K.L., Kwei, A.S., et al., Novel nanofiber-based scaffold for rotator cuff repair and augmentation. Tissue engineering. Part A, 2009. 15(1): p. 115-26.

9. Zioupos, P. and Barbenel, J.C., Mechanics of native bovine pericardium. I. The multiangular behaviour of strength and stiffness of the tissue. Biomaterials, 1994.15(5): p. 366-373.

10. Zioupos, P. and Barbenel, J.C., Mechanics of native bovine pericardium. II. A structure based model for the anisotropic mechanical behaviour of the tissue. Biomaterials, 1994. 15(5): p. 374-382.

**Claims**

1. A method of forming a matrix of aligned nanofibres having high porosity and pore size, the method comprising spraying a polymer solution towards a rotating drum so as to form nanofibres which are collected on the drum, such that the porosity of the nanofibrillar matrix is at least 95%, and the pore size of the nanofibrillar matrix is at least 50 μm.

2. The method of claim 1, wherein the spraying step comprises spraying a polymer solution under gas pressure through a nozzle.

3. The method of claim 1 or 2, wherein the diameter of the drum is at least 50 mm.

4. The method of claim 1, 2 or 3, wherein the drum is rotated at a speed of at least 8 m/s.

5. A method of forming an artificial tissue and comprising:

   forming a matrix of aligned nanofibres in accordance with the method of any of claims 1 to 4;
   removing the matrix from the drum; and
   depositing cells onto the matrix of aligned nanofibres and allowing the cells to form artificial tissue.

6. The method of claim 5, further comprising the step of functionalizing the matrix by binding bioactive molecules to the nanofibres, preferably wherein the bioactive molecules are peptides.

7. The method of claim 5, wherein the bioactive molecules are bound to the nanofibres via linkers.

8. The method of any one of claims 5 to 7, wherein the step of allowing the cells to form artificial tissue comprises culturing the cells.

9. The method of any one of claims 5 to 8, wherein the cells are adipose-derived mesenchymal stem cells, adipose-derived stem cells, fibroblasts, smooth muscle cells, endothelial cells, bone marrow derived stem cells, valve interstitial cells or myofibroblasts.

10. A matrix of aligned nanofibres having a porosity of at least 95%, and a median pore size of at least 50 μm.

11. An artificial tissue obtainable by or obtained by the method of any one of claims 5 to 10, preferably wherein the tissue is cardiac tissue.

12. Use of the artificial tissue of claim 11 for the production of an artificial heart valve; or for the production of an artificial pericardium.

13. The artificial tissue of claim 11 for use in the treatment of diseased or damaged tissue.

14. A method of treating diseased or damaged tissue, said method comprising administering the artificial tissue of claim 11 into a patient in need thereof.

15. The method of claim 14, wherein

   a) the diseased or damaged tissue has anisotropic organization; and/or.
   b) the diseased or damaged tissue is skin, ligaments, tendons, vessels, valves or muscles; and/or
   c) the disease is cardiovascular disease.

Figure 1

## 0 m/s

Figure 2a

## 5.8 m/s

Figure 2b

## 11.6 m/s

Figure 2c

## 17.5 m/s

Figure 2d

## 23.3 m/s

Figure 2e

Figure 3a

Figure 3b

Figure 4a

Figure 4b

Figure 4c

Figure 5

Figure 6b

Figure 6a

Europäisches
Patentamt

European
Patent Office

Office européen
des brevets

# EUROPEAN SEARCH REPORT

**Application Number**

EP 14 19 5230

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2010/081832 A1 (INST NAT SANTE RECH MED [FR]; SOHIER JEROME [FR]; LAYROLLE PIERRE [FR]) 22 July 2010 (2010-07-22)<br>* page 5, lines 22-23; figure 1 *<br>* page 6, line 12 *<br>* page 7, line 31 - page 8, line 7 * | 1-15 | INV.<br>A61L27/18<br>A61L27/54<br>A61L27/56 |
| A | US 2007/269481 A1 (LI SONG [US] ET AL) 22 November 2007 (2007-11-22)<br>* paragraphs [0003], [0005], [0147], [0162], [0172], [0189]; figures 2A,4A * | 1-15 | |
| A | EP 2 404 627 A1 (UNIV NANTES I [FR]; CHU NANTES [FR]) 11 January 2012 (2012-01-11)<br>* paragraph [0023]; claim 1 * | 1-15 | |

TECHNICAL FIELDS
SEARCHED       (IPC)

A61L

The present search report has been drawn up for all claims

| Place of search | Date of completion of the search | Examiner |
|---|---|---|
| The Hague | 16 March 2015 | Siebum, Bastiaan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 14 19 5230

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2015

| Patent document cited in search report | | Publication date | Patent family member(s) | | Publication date |
|---|---|---|---|---|---|
| WO 2010081832 | A1 | 22-07-2010 | CA | 2749328 A1 | 22-07-2010 |
| | | | EP | 2376692 A1 | 19-10-2011 |
| | | | ES | 2430353 T3 | 20-11-2013 |
| | | | US | 2012093912 A1 | 19-04-2012 |
| | | | WO | 2010081832 A1 | 22-07-2010 |
| US 2007269481 | A1 | 22-11-2007 | AU | 2007211018 A1 | 09-08-2007 |
| | | | CA | 2640601 A1 | 09-08-2007 |
| | | | EP | 1974015 A2 | 01-10-2008 |
| | | | EP | 2599858 A2 | 05-06-2013 |
| | | | JP | 5249785 B2 | 31-07-2013 |
| | | | JP | 2009524507 A | 02-07-2009 |
| | | | KR | 20080091827 A | 14-10-2008 |
| | | | US | 2007269481 A1 | 22-11-2007 |
| | | | WO | 2007090102 A2 | 09-08-2007 |
| EP 2404627 | A1 | 11-01-2012 | CA | 2804551 A1 | 12-01-2012 |
| | | | EP | 2404627 A1 | 11-01-2012 |
| | | | EP | 2590694 A2 | 15-05-2013 |
| | | | US | 2013138155 A1 | 30-05-2013 |
| | | | WO | 2012004407 A2 | 12-01-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010081832 A
- US 20070269481 A

### Non-patent literature cited in the description

- **PHAM QP ; SHARMA U ; MIKOS AG.** Electrospun poly(epsilon-caprolactone) microfiber and multilayer nanofiber/microfiber scaffolds: characterization of scaffolds and measurement of cellular infiltration. *Biomacromolecules.,* October 2006, vol. 7 (10), 2796-805
- **EKAPUTRA, A.K. ; PRESTWICH, G.D. et al.** Combining electrospun scaffolds with electrosprayed hydrogels leads to three-dimensional cellularization of hybrid constructs. *Biomacromolecules,* 2008, vol. 9 (8), 2097-2103
- **PHAM, Q.P. ; SHARMA, U. ; MIKOS, A.G.** Electrospun poly(epsilon-caprolactone) microfiber and multilayer nanofiber/microfiber scaffolds: characterization of scaffolds and measurement of cellular infiltration. *Biomacromolecules,* 2006, vol. 7 (10), 2796-805
- **ZHU, X. ; CUI, W. et al.** Electrospun Fibrous Mats with High Porosity as Potential Scaffolds for Skin Tissue Engineering. *Biomacromolecules,* 2008, vol. 9 (7), 1795-1801
- **HONG, J.K. ; MADIHALLY, S.V.** Three-dimensional scaffold of electrosprayed fibers with large pore size for tissue regeneration. *Acta biomaterialia,* 2010, vol. 6 (12), 4734-42
- **NISBET, D.R. ; FORSYTHE, J.S. et al.** Review paper: a review of the cellular response on electrospun nanofibers for tissue engineering. *Journal of biomaterials applications,* 2009, vol. 24 (1), 7-29
- **MARTINS, A. ; ARAUJO, J.V. et al.** Electrospun nanostructured scaffolds for tissue engineering applications. *Nanomedicine,* 2007, vol. 2 (6), 929-42
- **BAKER, B.M. ; SHAH, R.P. et al.** Sacrificial nanofibrous composites provide instruction without impediment and enable functional tissue formation. *Proceedings of the National Academy of Sciences of the United States of America,* 2012, vol. 109 (35), 14176-81
- **MOFFAT, K.L. ; KWEI, A.S. et al.** Novel nanofiber-based scaffold for rotator cuff repair and augmentation. *Tissue engineering. Part A,* 2009, vol. 15 (1), 115-26
- **ZIOUPOS, P. ; BARBENEL, J.C.** Mechanics of native bovine pericardium. I. The multiangular behaviour of strength and stiffness of the tissue. *Biomaterials,* 1994, vol. 15 (5), 366-373
- **ZIOUPOS, P. ; BARBENEL, J.C.** Mechanics of native bovine pericardium. II. A structure based model for the anisotropic mechanical behaviour of the tissue. *Biomaterials,* 1994, vol. 15 (5), 374-382